(19) Europäisches Patentamt
European Patent Office
Office européen des brevets

(11) EP 0 857 723 B1

(12) **EUROPÄISCHE PATENTSCHRIFT**

(45) Veröffentlichungstag und Bekanntmachung des Hinweises auf die Patenterteilung:
**09.01.2002 Patentblatt 2002/02**

(51) Int Cl.[7]: **C07D 317/70**, C11B 9/00

(21) Anmeldenummer: **98100936.8**

(22) Anmeldetag: **21.01.1998**

(54) **Cyclische Cedren-Acetale, ihre Herstellung und ihre Verwendung**

Cyclic cedrene acetals, their preparation and their use

Acétals cycliques du cedrène, leur préparation et leur utilisation

(84) Benannte Vertragsstaaten:
**BE CH DE ES FR GB IE LI NL**

(30) Priorität: **06.02.1997 DE 19704484**

(43) Veröffentlichungstag der Anmeldung:
**12.08.1998 Patentblatt 1998/33**

(73) Patentinhaber: **Dragoco Gerberding & Co Aktiengesellschaft**
**37603 Holzminden (DE)**

(72) Erfinder:
- **Pickenhagen, Wilhelm, Dr.**
**37671 Höxter (DE)**
- **Schatkowski, Dietmar**
**37627 Stadtoldendorf (DE)**

(74) Vertreter: **Eisenführ, Speiser & Partner**
**Martinistrasse 24**
**28195 Bremen (DE)**

(56) Entgegenhaltungen:
- **STEFFEN ARCTANDER : "Perfume and Flavor Chemicals (Aroma Chemicals) I" 1969 , STEFFEN ARCTANDER , MONTCLAIR, N.J., U.S.A. XP002064713 * compounds 593 - 603 ***

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

EP 0 857 723 B1

## Beschreibung

Seit tausenden von Jahren haben die starken Geruchseigenschaften von tierischen Sekreten, Drüsen und anderen Organteilen den Menschen in seinen Bann gezogen. Während man anfänglich den ausströmenden Düften magische Kräfte zuordnete und diese daher für religiöse und kulturelle Zwecke einsetzte, kamen seit dem Altertum verschiedene andere Anwendungsformen, z. B. als Arzneimittel hin. Zu Beginn dieses Jahrhunderts hielten Ambra, Moschus und Zibet Einzug als kostbare Ingredienzen in der Parfümerie. So erfolgten erste analytische Arbeiten zur Strukturaufklärung des riechenden Prinzips bei der Ambra Mitte der 20er Jahre. Zu Beginn der 30er Jahre erfolgten dann erste synthetische Arbeiten zu ihrer Herstellung im technischen Maße. Der steigende Bedarf an Riechstoffen konnte durch Produkte natürlichen, pflanzlichen und tierischen Ursprungs nicht mehr gedeckt werden. Die Deckung des steigenden Bedarfs an Riechstoffen mit verbesserten Eigenschaften wie Geruchsqualität, Stabilität in technischen Anwendungen, Hautverträglichkeit, Umweltverträglichkeit sowie Haftung ist nur durch chemische Synthese von Riechstoffen möglich.

Aufgrund einer ausgewogenen Ökobilanz sind Produkte anzustreben, die zum einen nach Möglichkeit aus nachwachsenden Rohstoffen und zum anderen biologisch abbaubar sind. Ein solcher in großen Mengen verfügbarer Rohstoff natürlichen Ursprungs ist das in verschiedenen Zedemholzarten vorkommende Cedren.

Bereits vor zwei Jahrzehnten wurden eine Reihe von Folgeprodukten des (-α)-Cedren (1) hergestellt und Riechstoffeigenschaften beschrieben. G. Ohloff berichtet in seinem Buch "Riechstoff und Geruchsinn" (Springer-Verlag, Berlin 1990, ISD-N 3-540-52560-2, Seiten 170 - 172) zusammenfassend, daß sich vom α-Cedren (1) eine Reihe kommerzielle Riechstoffe ableiten. S. Arctander beschreibt in "Perfume and Flavor Chemicals" (Nr. 593-600, 602) mehrere sich vom Cedren ableitende Riechstoffe.

Die aus α-Cedren (**1**) durch Epoxidation, Allyloxidation, Veresterung und Veretherung zugänglichen Derivate gelten als sensorisch wertvolle Riechstoffe vom warm-holzigen Geruchstyp, zum Teil mit ambraartigem Effekt.

Entsprechend dem beschriebenen Stand der Technik gilt dieses Gebiet der Riechstoffchemie als besonders gut untersucht. Neben einigen Cedren-Derivaten mit Riechstoffeigenschaften, sind eine größere Zahl von Derivaten der genannten Sesquiterpene bekannt, die keinen oder keine wesentlichen olfaktorischen Eigenschaften besitzen. Es ist aus diesem Grund besonders überraschend, daß nunmehr auf dem Gebiet der Cedren-Derivate die hier beschriebenen neuen Verbindungen der allgemeinen Formel **A** (in der die geschlängelten Linien α- und β-Konfiguration sowie $R_1$ und $R_2$ Wasserstoff- bzw. Methyl-, Ethyl-, Propyl-, Butyl-, Isobutyl-, PentylIsopentyl-, Hexyl-, Isohexyl-, Cyclopentyl- und Cyclohexyl-Reste bedeuten) aufgefunden werden konnten, die über ganz eigentümliche geruchliche Eigenschaften verfügen und sich hierin deutlich von den bekannten Riechstoffen aus α-Cedren (**1**) abheben und diese übertreffen. Die Cedren-Derivate der allgemeinen Formel **A** besitzen Geruchseigenschaften vom Ambratyp und vermitteln gleichzeitig einen strahlenden, starken Effekt, der ganz unterschiedliche Parfümnoten verstärkt und ihre Duftwirkung verlängert.

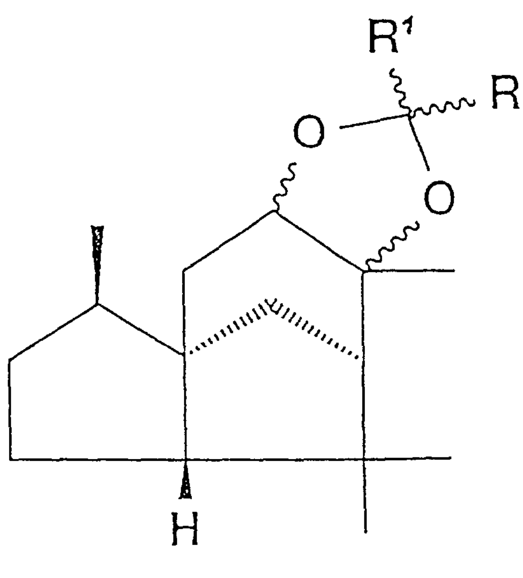

(A)

Zur Herstellung der Verbindung der allgemeinen Formel **A** wurde (-)-α-Cedren (**1**) in bekannter Weise durch Behandeln mit Peressigsäure (Organikum, Organisch-Chemisches Grundpraktikum, VEB Deutscher Verlag der Wissenschaften, Berlin, 1986, Best.-Nr. 5714576, Seite 568) zu (-)-α-Cedrenepoxid (**2**) umgesetzt. Das aus Cedren erhaltene Epoxid (**2**) wurde dann in bekannter Weise (Houben-Weyl) in ein Gemisch der epimeren Diole (**3**) überführt, wobei dieses Diolgemisch in Abhängigkeit von den Produktionsbedingungen unterschiedliche Isomerenverteilungen und Geruchseffekte aufweist. Die Strukturzuordnung erfolgte aufgrund der NMR-Meßergebnisse.

Die neuen cyclischen Acetale der allgemeinen Formel **A** werden aus den Diolen 3, die in reiner bzw. in angereicherter Form oder als Gleichgewichtsgemisch vorlagen, in an sich bekannter Weise durch Umsetzung mit aliphatischen Aldehyden und Ketonen unter Säurekatalyse unter Verwendung verschiedener Lösungsmittel, beispielsweise Toluol, Cyclohexan, Benzin-Fraktionen, Diethylether hergestellt.

Grafik 1

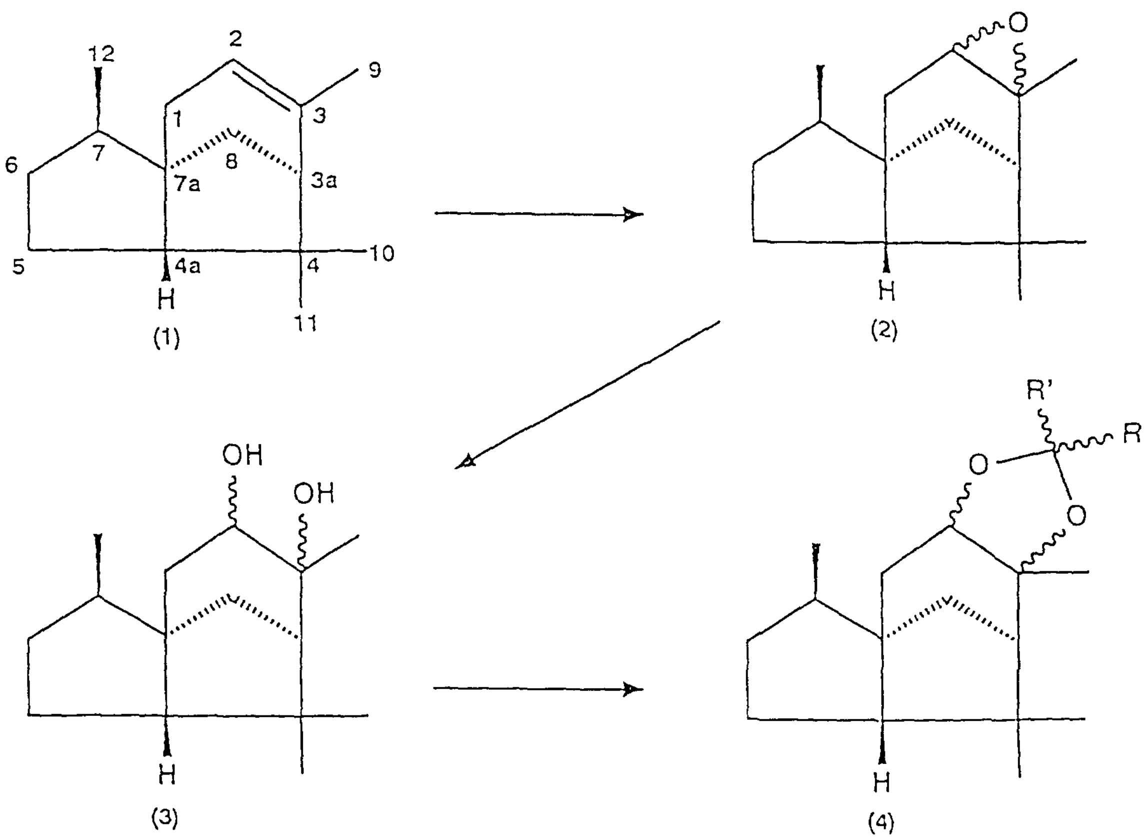

(1) (2) (3) (4)

Diese epimerenreinen Epoxide **2a** und **2b** lassen sich einzeln oder im Gemisch in Abhängigkeit von den Produktionsbedingungen zu den diastereomeren Diolen **3a - f** überführen. In Abhängigkeit von gewählten Reaktionsbedingungen können hierbei die epimeren Epoxide **2a** und **2b** in unterschiedlichen Mengenverhältnissen anfallen, so daß nach Öffnung der Epoxide zu den diastereomeren Diolen **3a/b/c/d/e/f/g/h** diese ebenfalls in unterschiedlichen Mengenverhältnissen vorliegen. Die stereochemischen Verhältnisse bleiben bei der Umsetzung mit den Aldehyden und Ketonen im wesentlichen unverändert, so daß die neuen gemischten Acetale der Formel **A** in diesem Fall als Diastereomerengemische vorliegen.

Die neuen gemischten Acetale der Formel **A** besitzen jeweils in reiner Form als auch als Stereoisomerengemische originelle Riechstoffeigenschaften und können in reiner Form oder als Diastereomerengemische vorteilhaft als Riechstoffe bzw. Bestandteile von Parfümölen eingesetzt werden.

Grafik 2

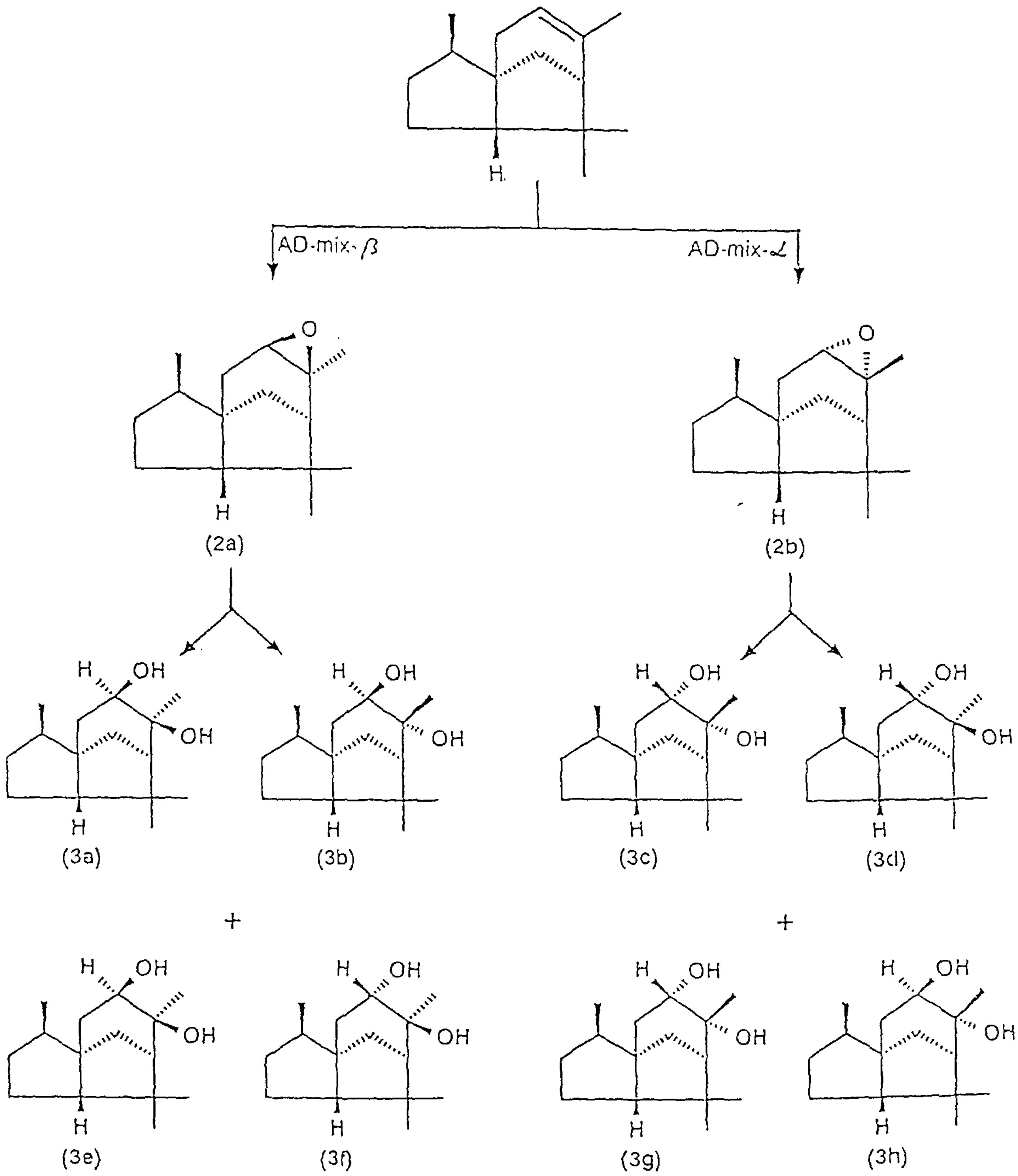


(2a) (2b)

(3a) (3b) (3c) (3d)

\+ +

(3e) (3f) (3g) (3h)

**Beispiel 1**

Herstellung von (-)-α-Cedrenepoxid (2)

In einem 2 l-Rührwerk mit Rückflußkühler, Thermometer und Tropftrichter wurden 500 g (2,00 mol) (-)-α-Cedren (**1**) (82 %ig nach GC $[\alpha]_D$= - 75,8 °), 500 ml Diethylether, 100 g Natriumacetat vorgelegt und anschließend bei 20 - 25 °C innerhalb von 1 Std. mit 430 g (2,20 mol) Peressigsäure 40 %ig versetzt. Nach vollendetem Zutropfen wurden 2 Std. nachgerührt und anschließend aufgearbeitet. Die abgetrennte organische Phase wurde mit Sodalösung und Wasser neutralgewaschen, über Natriumsulfat getrocknet und das Lösungsmittel unter reduziertem Druck abdestilliert. Es verblieben 526,5 g Rohprodukt (78,2 %ig nach GC).
Gaschromatogramm (HP 5970 B, DBWAX-60 N, 60 m, 60 °C- 240 °C, 4 °C/min.).
Eine Destillation von 100 g Rohprodukt über eine 15 cm Vigreux-Kolonne ergab 95 g rohes **2**; $Kp_{2mm}$: 115 °C bis 138 °C, GC **2** (80,2 %).

D 20/4: 1.0009
n 20/D: 1.4965
[α] 20/D: -72.4

**Beispiel 2**

Herstellung von 2β-, 3β-Epoxicedren (**2a**) - Grafik 2

In einem 250 ml-Rührwerk mit Rückflußkühler, Thermometer und Tropftrichter wurden 50 ml tert-Butanol, 50 ml Wasser und 14 g AD-mix-β (= 0,01 mol Äquivalent Olefin; Fa. Aldrich) vorgelegt und 2 Std. bei Raumtemperatur gerührt.
Nach dieser Zeit hatten sich zwei Phasen gebildet, welche sich klar von einander trennten. Dabei erschien die untere Phase leicht gelb. Nach dieser Zeit wurden zu der gerührten Lösung 0,95 g (0,01 mol) Methansulfonsäureamid zugegeben. Anschließend wurde auf 0 °C heruntergekühlt. Nachdem eines der vorher gelösten Salze ausgefallen war, tropfte man bei 0 °C unter starkem Rühren 2,55 g (0,01 mol) (-)-α-Cedren (**2**) (82,0 %ig nach GC) zu und rührte bei dieser Temperatur insgesamt 60 Std. nach. Nach dieser Zeit versetzte man das Reaktionsgemisch mit wässeriger Natriumsulfitlösung, ließ auf Raumtemperatur erwärmen und rührte insgesamt noch 1 Std. bei dieser Temperatur nach. Nach dem Versetzen mit Ether wurde die organische Phase abgetrennt, die wässerige Phase 3 mal mit jeweils 50 ml Diethylether extrahiert, die vereinigten organischen Phasen einmal mit 10 %iger KOH und einmal mit Wasser neutralgewaschen. Anschließend wurde über Natriumsulfat getrocknet und das Lösungsmittel unter vermindertem Druck abdestilliert.
Es verblieben 1,9 g Rohprodukt. GC **2a** (78 %).

**Beispiel 3**

Herstellung von 2α-, 3α-Epoxicedren (**2b**) - Grafik 2

In einem 250 ml Rührwerk mit Rückflußkühler, Thermometer und Tropftrichter wurden 50 ml tert-Butanol, 50 ml Wasser und 14 g AD-mix-α (= 0,01 mol Äquivalent Olefin; Fa. Aldrich) vorgelegt und insgesamt 1 Std. bei Raumtemperatur gerührt. Nach dieser Zeit hatten sich zwei Phasen gebildet. Dabei erschien die untere Phase leicht gelb. Nach dieser Zeit gab man zu der gerührten Lösung insgesamt 0,95 g (0,01 mol) Methansulfonsäureamid. Anschließend wurde auf 0 °C heruntergekühlt. Nachdem eines der gelösten Salze ausgefallen war, tropfte man bei 0 °C unter starkem Rühren zu dieser Suspension 2,55 g (0,01 mol) (-)-α-Cedren (**2**) (82,0 %ig nach GC) zu und rührte insgesamt bei dieser Temperatur 72Std. nach. Nach dieser Zeit versetzte man das Reaktionsgemisch mit wässeriger Natriumsulfit-Lösung, ließ auf Raumtemperatur erwärmen und rührte insgesamt 1 h bei Raumtemperatur nach. Anschließend wurde aufgearbeitet. Nach dem Versetzen mit Ether wurde die organische Phase abgetrennt, die wässerige Phase dreimal mit jeweils 50 ml Diethylether extrahiert, die vereinigten organischen Phasen einmal mit 10 %iger KOH und einmal mit Wasser neutralgewaschen. Anschließend wurde das Reaktionsgemisch über Natriumsulfat getrocknet und das Lösungsmittel unter vermindertem Druck abdestilliert.
Es verblieben 1,8 g Rohprodukt; GC: **2b** (84,1 %)

**Beispiel 4**

Herstellung von Cedran-Diol-Gemisch (**3**) - Grafik 1

In einem 4 l-Dreihalskolben mit Rückflußkühler, Tropftrichter und Thermometer wurden 2000 g 10 %ige Schwefelsäure und 3 g Aliquat R336 vorgelegt. Anschließend wurden unter starkem Rühren innerhalb von 10 Minuten 300 g (1,09 mol) (-)-$\alpha$-Cedrenepoxid (**2**) (80,2 %ig nach GC) aus Beispiel 2 zugetropft und anschließend insgesamt 48 Std. bei 20 - 25 °C unter starkem Rühren nachgerührt. Das ausgefallene organische Produkt wurde anschließend abfiltriert, mit 2 %iger Sodalösung und Wasser neutralgewaschen. 120 g abgetrenntes DiolGemisch (**3**) wurden anschließend aus 2 l Cyclohexan umkristallisiert. Nach dem Absaugen des Lösungsmittels verblieben 98 g kristallines Cedran-Diol **3** (37,6 % d. Th.).

Smp. 167 - 168 °C
GC: Bedingungen siehe Beispiel 1
**3a, e, c, g:** 49,3 %
**3b, f, d, h:** 32,0 %
GC/MS: HP 5970 B, DBWAX-60 N, 60 m, 60 °C - 240 °C, 4 °C/min.
**3a, e, c, g:** $R_t$ = 55,60
MS: m/e (%) = 238 (10, $M^+$), 223 (45), 205 (34), 193 (30), 167 (46), 121 (46), 107 (61), 99 (66), 81 (58), 43 (100).
**3b, d, f, h:** $R_t$ = 55,97
MS: m/e (%) = 238 (10, $M^+$), 223 (38), 205 (29), 193 (20), 167 (34), 121 (44), 109 (37), 107 (40), 81 (46), 43 (100).

**Beispiel 5**

Umsetzung von Cedran-Diol-Gemisch **3** (aus Grafik 1) mit Aceton

In einem 250 ml-Rührwerk mit Tropftrichter, Thermometer und Rückflußkühler wurden 10,1 g (0,04 mol) Cedran-Diol-Gemisch **3** (Grafik 1, aus Beispiel 4), 23,3 g (0,4 mol) Aceton, 100 ml Toluol und 0,2 g p-Toluolsulfonsäure insgesamt 24 Std. bei 20 - 25 °C gerührt. Nach dieser Zeit wurde einmal mit 10 ml 10 %iger Sodalösung und einmal mit 50 ml Wasser neutralgewaschen, über Natriumsulfat getrocknet und das Lösungsmittel unter vermindertem Druck abdestilliert. Es verblieben 10,7 g kristallines Rohprodukt.

Das kristalline Rohprodukt wurde aus 100 ml Acetessigester umkristallisiert. Nach dem Absaugen des Lösungsmittels verblieben 5,6 g weißes, kristallines Produkt.

GC (Bedingungen siehe Beispiel 1): 95,6 %ig
Smp.: 1 57 -1 58 °C
GC/MS: Bedingungen siehe Beispiel 2
$R_t$ = 41,43
MS: m/e (%) = 278 (5, $M^+$), 263 (49), 221 (100), 203 (48), 161 (10), 147 (21), 133 (41), 119 (73), 105 (36), 69 (35), 43 (51).
$^{13}$C-NMR ($CDCl_3$), Varian VXR 300: $\delta$ [ppm] = 15.42, 27.56, 28.74, 29.67, 30.26, 31.12 ($\underline{C}H_3$), 25.42, 35.88, 38.51, 41.00 ($\underline{C}H_2$), 41.92, 57.39, 58.58, 78.79 ($\underline{C}$H), 42.43, 52.38, 84.99, 108.87 ($\underline{C}$).

**Beispiel 6**

Umsetzung von Cedran-Diol-Gemisch **3** (aus Grafik 1) mit Methyl-Ethyl-Keton

In einem 250 ml-Rührwerk mit Tropftrichter, Rückflußkühler und Thermometer wurden 10,1 g (0,04 mol) Cedran-Diol-Gemisch **3** (aus Beispiel 4), 28,8 g (0,4 mol) Methyl-Ethyl-Keton, 100 ml Toluol und 0,2 g p-Toluolsulfonsäure, insgesamt 48 Std. bei 20 - 25 °C gerührt. Nach dieser Zeit wurde einmal mit 10 %iger Sodalösung und einmal mit Wasser neutralgewaschen, über Natriumsulfat getrocknet und das Lösungsmittel unter vermindertem Druck abdestilliert. Es verblieben 10,5 g Rohprodukt.

GC (Bedingungen siehe Beispiel 1): $R_t$ = 44,5 = 15,1 %
$R_t$ = 45,4 =19,1%
GC/MS (Bedingungen siehe Beispiel 2)
$R_t$ = 44,57
MS: m/e (%) = 292 (3, $M^+$), 277 (4), 263 (34), 221 (100), 203 (49), 133 (45), 119 (82), 105 (40), 69 (40), 43 (50).

$R_t$ = 45,20
MS: m/e (%) = 292 (2, $M^+$), 277 (2), 263 (35), 221 (100), 203 (52), 133 (43), 119 (76), 105 (38), 69 (37), 43 (50).

**Beispiel 7**

Umsetzung von Cedran-Diol-Gemisch **3** (aus Grafik 1) mit Methyl-Propyl-Keton

In einem 250 ml-Rührwerk mit Thermometer und Rückflußkühler wurden 10,1 g (0,04 mol) Cedran-Diol-Gemisch **3** (Grafik 1 aus Beispiel 4), 34,4 g (0,4 mol) Methyl-Propyl-Keton, 100 ml Toluol und 0,2 g p-Toluolsulfonsäure insgesamt 24 Std. bei 20 - 25 °C gerührt. Nach dieser Zeit wurde einmal mit 10 %iger Sodalösung und einmal mit Wasser gewaschen, über Natriumsulfat getrocknet und das Lösungsmittel unter vermindertem Druck abdestilliert. Es verblieben 10,2 g hellgelbes, kristallines Rohprodukt.

GC (Bedingungen siehe Beispiel 2)
$R_t$ = 44,10 = 9,2 %
$R_t$ = 44,68 = 17,7 %
GC/MS: Bedingungen siehe Beispiel 2
$R_t$ = 45,20
MS: m/e (%) = 306 (2, $M^+$), 291 (3), 263 (33), 221 (100), 203 (41), 147 (19), 133 (38), 119 (68), 105 (34), 69 (35), 43 (52).
$R_t$ = 44,68
MS: m/e (%) = 306 (1, $M^+$), 291 (1), 263 (31), 221 (100), 203 (45), 147 (19), 133 (39), 119 (70), 105 (34), 69 (34), 43 (50).

**Beispiel 8**

Umsetzung von Cedran-Diol-Gemisch **3** (Grafik 1) mit Diethylketon

In einem 250 ml-Rührwerk mit Rückflußkühler und Thermometer wurden 10,1 g (0,04 mol) Cedran-Diol-Gemisch **3** (Grafik 1 aus Beispiel 4), 34,4 g (0,4 mol) Diethylketon, 100 ml Toluol und 0,2 g p-Toluolsulfonsäure insgesamt 24 Std. bei 20 - 25 °C gerührt. Nach dieser Zeit wurde einmal mit 10 %iger Sodalösung und einmal mit Wasser gewaschen, über Natriumsulfat getrocknet und das Lösungsmittel unter vermindertem Druck abdestilliert. Es verblieben 10,8 g hellbraunes, kristallines Rohprodukt.

GC (Bedingungen siehe Beispiel 1): 29,5 %
GC/MS (Bedingungen siehe Beispiel 2)
$R_t$ = 44,61
MS: m/e (%) = 306 (4, $M^+$), 291 (1), 277 (15), 221 (39), 203 (21), 177 (31), 159 (37), 119 (44), 91 (48), 69 (100), 41 (85).

**Beispiel 9**

Umsetzung von Cedran-Diol-Gemisch **3** (Grafik 1) mit Cyclopentanon

In einem 250 ml-Rührwerk mit Rückflußkühler und Thermometer wurden 10,1 g (0,04 mol) Cedran-Diol-Gemisch **3** (Grafik 1 - aus Beispiel 4), 33,6 g (0,4 mol) Cyclopentanon, 100 ml Toluol und 0,2 g p-Toluolsulfonsäure 24 Std. bei 20 - 25 °C gerührt. Nach dieser Zeit wurde einmal mit 10 %iger Sodalösung und einmal mit 50 ml Wasser gewaschen, über Natriumsulfat getrocknet und das Lösungsmittel unter vermindertem Druck abdestilliert. Es verblieben 10,5 g gelbes, kristallines Rohprodukt.

GC (Bedingungen siehe Beispiel 1): 49,8 %
GC/MS (Bedingungen siehe Beispiel 2)
$R_t$ = 49,92
MS: m/e (%) = 304 (15, $M^+$), 275 (22), 221 (100), 205 (88), 133 (46), 119 (90), 105 (48), 69 (54), 55 (69), 41 (54).

**Beispiel 10**

Umsetzung von Cedran-Diol-Gemisch **3** (Grafik 1) mit Cyclohexanon

In einem 250 ml-Rührwerk mit Rückflußkühler und Thermometer wurden 10,1 g (0,04 mol) Cedran-Diol-Gemisch **3** (Grafik 1 - aus Beispiel 4), 39,2 g (0,4 mol) Cyclohexanon, 100 ml Toluol und 0,2 g p-Toluolsulfonsäure 24 Std. bei 20 - 25 °C gerührt. Anschließend wurde einmal mit 10 %iger Sodalösung und einmal mit Wasser neutralgewaschen, über Natriumsulfat getrocknet und das Lösungsmittel unter vermindertem Druck abdestilliert. Es verblieben 11,3 g hellgelbes Rohprodukt.

GC (Bedingungen siehe Beispiel 1): 73 %
GC/MS (Bedingungen siehe Beispiel 2)
$R_t$ = 53,22
MS: m/e (%) = 318 (22, $M^+$), 275 (24), 221 (100), 203 (63), 147 (25), 133 (52), 119 (91), 105 (49), 69 (68), 55 (66), 41 (61).
$^{13}$C-NMR ($CDCl_3$), Varian VXR-300: $\delta$ [ppm] = 15.45, 28.10, 28.71, 31.20 ($\underline{C}H_3$), 24.11, 24.25, 25.17, 25.37, 35.78, 38.48, 38.92, 39.57, 41.71 ($\underline{C}H_2$), 41.87, 57.40, 58.66, 78.21 ($\underline{C}H$), 42.83, 52.42, 84.36, 109.83 ($\underline{C}$).

**Beispiel 11**

Umsetzung von Cedran-Diol-Gemisch **3** (Grafik 1) mit Acetaldehyd

In einem 250 ml-Rührwerk mit Rückflußkühler und Thermometer wurden 10,1 g (0,04 mol) Cedran-Diol-Gemisch **3** (Grafik 1 - aus Beispiel 4), 17,6 g (0,4 mol) Acetaldehyd, 100 ml Toluol und 0,2 g p-Toluolsulfonsäure insgesamt 3 Std. bei 20 - 25 °C gerührt. Nach dieser Zeit wurde einmal mit 10 %iger Sodalösung und einmal mit Wasser gewaschen, über Natriumsulfat getrocknet und das Lösungsmittel unter vermindertem Druck abdestilliert. Es verblieben 10,3 g helles Rohprodukt.

GC (Bedingungen siehe Beispiel 1): 37,8 %, 60,08 %
GC/MS (Bedingungen siehe Beispiel 2)
$R_t$ = 41.29
MS: m/e (%) = 264 (19, $M^+$), 249 (43), 221 (18), 203 (77), 177 (65), 119 (100), 105 (61), 95 (58), 69 (71), 43 (100).
$R_t$ = 40.91
MS: m/e (%) = 264 (36, $M^+$), 249 (49), 221 (24), 203 (83), 177 (30), 119 (91), 105 (57), 95 (60), 69 (69), 43 (100).

**Beispiel 12**

Umsetzung von Cedran-Diol-Gemisch **3** (Grafik 1) mit Propionaldehyd

In einem 250 ml-Rührwerk mit Rückflußkühler und Thermometer wurden 10,1 g (0,04 mol) Cedran-Diol-Gemisch **3** (Grafik 1 - aus Beispiel 4), 23,2 g (0,4 mol) Propionaldehyd, 100 ml Toluol und 0,2 g p-Toluolsulfonsäure insgesamt 4 Std. bei 20 - 25 °C gerührt. Nach dieser Zeit wurde einmal mit 10 %iger Sodalösung und einmal mit Wasser gewaschen, über Natriumsulfat getrocknet und das Lösungsmittel unter vermindertem Druck abdestilliert. Es verblieben 11,2 g helles, kristallines Rohprodukt.

GC (Bedingungen siehe Beispiel 1): 25,7 %, 62,3 %
GC/MS (Bedingungen siehe Beispiel 2)
$R_t$ = 42,49
MS: m/e (%) = 278 (20, $M^+$), 249 (31), 203 (100), 161 (15), 147 (32), 133 (63), 119 (97), 105 (53), 91 (24), 69 (54), 43 (37).
$R_t$ = 42,94
MS: m/e (%) = 278 (4, $M^+$), 249 (33), 203 (100), 161 (15), 147 (33), 133 (64), 119 (98), 105 (54), 91 (24), 69 (54), 41 (42).

**Beispiel 13**

Umsetzung von Cedran-Diol-Gemisch **3** (Grafik 1) mit Butyraldehyd

In einem 250 ml-Rührwerk mit Rückflußkühler und Thermometer wurden 10,1 g (0,04 mol) Cedran-Diol-Gemisch **3** (Grafik 1 - aus Beispiel 4), 28,8 g (0,4 mol) Butyraldehyd, 100 ml Toluol und 0,2 g p-Toluolsulfonsäure 6 Std. bei 20 - 25 °C gerührt. Nach dieser Zeit wurde einmal mit 10 %iger Sodalösung und einmal mit 50 ml Wasser gewaschen, über Natriumsulfat getrocknet und das Lösungsmittel unter vermindertem Druck abdestilliert. Es verblieben 13,4 g helles Rohprodukt.

GC (Bedingungen siehe Beispiel 1): 26,3 %, 63,7 %
GC/MS (Bedingungen siehe Beispiel 2)
$R_t$ = 44,14
MS: m/e (%) = 292 (1, $M^+$), 249 (28), 203 (100), 161 (14), 147 (31), 133 (64), 119 (98), 105 (51), 69 (55), 43 (42).
$R_t$ = 44,69
MS: m/e (%) = 292 (3, $M^+$), 249 (29), 203 (100), 161 (13), 147 (31), 133 (59), 119 (89), 105 (47), 69 (54), 41 (44).

**Beispiel 14**

Umsetzung von Cedran-Diol-Gemisch **3** (Grafik 1) mit Isobutyraldehyd

In einem 250 ml-Rührwerk mit Rückflußkühler und Thermometer wurden 10,1 g (0,04 mol) Cedran-Diol-Gemisch **3** (Grafik 1 aus Beispiel 4), 28,8 g (0,4 mol) Isobutyraldehyd, 100 ml Toluol und 0,2 g p-Toluolsulfonsäure 5 Std. bei 20 - 25 °C gerührt. Nach dieser Zeit wurde einmal mit 10 %iger Sodalösung und einmal mit 50 ml Wasser gewaschen, über Natriumsulfat getrocknet und das Lösungsmittel unter vermindertem Druck abdestilliert. Es verblieben 10,2 g helles Rohprodukt.

GC (Bedingungen siehe Beispiel 1): 30,68 %, 61,32 %
GC/MS (Bedingungen siehe Beispiel 2)
$R_t$ = 42,29
MS: m/e (%) = 292 (1, $M^+$), 249 (31), 203 (100), 161 (13), 147 (29), 133 (55), 119 (87), 105 (43), 91 (17), 69 (42), 41 (29).
$R_t$ = 42,82
MS: m/e (%) = 291 (5, $M^+$), 249 (28), 203 (100), 161 (13), 147 (29), 133 (57), 119 (89), 105 (46), 91 (17), 69 (43), 41 (32).

**Beispiel 15**

Umsetzung von Cedran-Diol-Gemisch **3** (Grafik 1) mit Valeraldehyd

In einem 250 ml-Rührwerk mit Rückflußkühler und Thermometer wurden 10,1 g (0,04 mol) Cedran-Diol-Gemisch **3** (Grafik 1 - aus Beispiel 4), 34,4 g (0,4 mol) Valeraldehyd, 100 ml Toluol und 0,2 g p-Toluolsulfonsäure 7 Std. bei 20 - 25 °C gerührt. Nach dieser Zeit wurde einmal mit 10 %iger Sodalösung und einmal mit 50 ml Wasser gewaschen, über Natriumsulfat getrocknet und das Lösungsmittel unter vermindertem Druck abdestilliert. Es verblieben 10,6 g helles Rohprodukt.

GC (Bedingungen siehe Beispiel 1): 43,91 %, 16,67 %
GC/MS (Bedingungen siehe Beispiel 2)
$R_t$ = 46,39
MS: m/e (%) = 306 (1, $M^+$), 249 (27), 203 (100), 147 (31), 133 (59), 119 (85), 105 (47), 91 (24), 69 (52), 41 (46).
$R_t$ = 47,11
MS: m/e (%) = 306 (3, $M^+$), 249 (31), 203 (100), 147 (26), 133 (53), 119 (77), 105 (38), 91 (16), 69 (42), 41 (34).

**Beispiel 16**

Umsetzung von Cedran-Diol-Gemisch **3** (Grafik 1) mit Isovaleraldehyd

In einem 250 ml-Rührwerk mit Rückflußkühler und Thermometer wurden 10,1 g (0,04 mol) Cedran-Diol-Gemisch **3** (nicht umkristallisiert), 33,6 g (0,4 mol) Cyclopentanon, 100 ml Toluol und 0,2 g p-Toluolsulfonsäure 4 Std. bei 20 - 25 °C gerührt. Nach dieser Zeit wurde einmal mit 10 %iger Sodalösung und einmal mit 50 ml Wasser gewaschen, über Natriumsulfat getrocknet und das Lösungsmittel unter vermindertem Druck abdestilliert. Es verblieben 11 g farbloses Rohprodukt.

GC (Bedingungen siehe Beispiel 1): 16,4 %, 19,5 %, 12,2 % , 9,2 %, 9,5 %
GC/MS (Bedingungen siehe Beispiel 2)
$R_t$= 44,49
MS: m/e (%) = 306 (1, $M^+$), 249 (26), 203 (100), 161 (13), 147 (28), 133 (59), 119 (88), 105 (43), 69 (49), 41 (41).
$R_t$ = 45,1
MS: m/e (%) = 306 (3, $M^+$), 249 (30), 203 (100), 147 (26), 133 (54), 119 (79), 105 (40), 69 (43), 41 (37).
$R_t$ = 46,60
MS: m/e (%) = 306 (8, $M^+$), 291 (11), 263 (41), 220 (90), 203 (30), 177 (45), 150 (100), 121 (39), 69 (54), 43 (94).
$R_t$ = 48,47
MS: m/e (%) = 306 (7, $M^+$), 291 (10), 263 (14), 220 (81), 203 (30), 177 (42), 150 (100), 121 (39), 69 (49), 43 (85).
$R_t$= 48,80
MS: m/e (%) = 306 (10, $M^+$), 291 (11), 263 (11), 220 (77), 203 (18), 177 (40), 150 (100), 121 (41), 69 (44), 43 (85).

**Beispiel 17**

Umsetzung von Cedran-Diol-Gemisch (**3**) (Grafik 1) mit Formaldehyd

In einem 250 ml-Rührwerk mit Rückflußkühler und Thermometer wurden 30 g (1 mol) p-Formaldehyd, 200 ml Benzin und 3 g Schwefelsäure conc. zunächst 10 Minuten bei 20 - 25 °C gerührt. Dann gab man portionsweise innerhalb von 5 Minuten 5 g (0,019 mol) Cedran-Diol **3** (Grafik 1 aus Beispiel 4) zu. Anschließend wurde über einen Zeitraum von 3 Std. bei 20 - 25 °C gerührt. Nach dem Abfiltrieren vom p-Formaldehyd wurde das Reaktionsgemisch einmal mit 10 %iger Sodalösung und einmal mit Wasser gewaschen, über Natriumsulfat getrocknet und das Lösungsmittel unter vermindertem Druck abdestilliert. Es verblieben 5,4 g farbloses Rohgemisch.

GC (Bedingungen siehe Beispiel 1): 41,5 %
GC/MS (Bedingungen siehe Beispiel 2)
$R_t$ = 42,20
MS: m/e (%) =250 (31, $M^+$), 235 (37), 204 (37), 161 (47), 149 (50), 119 (48), 107 (59), 100 (100), 69 (63), 43 (96).

**Beispiel 18**

| **Akkord Muguet** | |
|---|---|
| Acetonid (aus Beispiel 5) | 2 |
| Aldehyd C11en 1 % | 10 |
| Citronellylacetat | 10 |
| Florindal (a) | 10 |
| cis-3-Hexenylacetat 10 % | 10 |
| Phenylethylisobutyrat | 15 |
| cis-3-Hexenol 1 % | 15 |
| Aldehyd C10 1 % | 15 |
| Indol 10 % | 20 |
| Phenylethyldimethylcarbinol | 25 |
| Sandranol (a) | 25 |
| Geraniol | 35 |
| Aldehyd C9 1 % | 40 |
| Hedione® (b) | 60 |

(a) = DRAGOCO
(b) = Firmenich

(fortgesetzt)

| Akkord Muguet | |
|---|---|
| Linalol | 60 |
| Phenylethylacetat | 60 |
| Lyral® (c) | 70 |
| Benzylacetat | 80 |
| Citronellol | 120 |
| Phenylethylalcohol | 140 |
| alpha-Hexylzimtaldehyd | 178 |
| | 1000 |

(c) = IFF

Die Zugabe von Acetonid (aus Beispiel 5) verbessert die Ausstrahlung und Frische der Kopfnote und erhöht die Haftung des Nachgeruchs.

**Beispiel 19**

| Akkord Jasmin | |
|---|---|
| Acetonid (aus Beispiel 5) | 1 |
| Methyloctincarbonat 1 % | 5 |
| Aldehyd C16 10 % | 5 |
| Kreosol 10 % (a) | 5 |
| Dimethylanthranilat 10 % | 5 |
| cis-3-Hexenol 10 % | 5 |
| Indol | 5 |
| Aldehyd C18 | 5 |
| Phenoxyethylisobutyrat | 5 |
| Kresol para 10 % | 10 |
| Maltol 1 % | 10 |
| Eugenol | 10 |
| cis-3-Hexenylacetat 10 % | 10 |
| Aldehyd C14 | 10 |
| Ethyllaurat | 10 |
| Methylanthranilat 10 % | 10 |
| Hedione® (b) | 15 |
| Hexylbenzoat | 15 |
| Cycloamylone (a) | 20 |
| Linalol | 30 |
| Benzylalkohol | 50 |
| Lactoscaton (a) | 60 |
| Benzylbenzoat | 149 |
| alpha-Hexylzimtaldehyd | 250 |
| Benzylacetat | 300 |
| | 1000 |

(a) = DRAGOCO
(b) = Firmenich

The Verwendung von Acetonid (aus Beispiel 5) verstärkt die typisch animalische, blumige Indolnote von Jasmin und verbessert die Ausstrahlung und die Haftung des Nachgeruchs.

## Patentansprüche

1. Cyclische Cedren-Acetale der allgemeinen Formel **A**, wobei geschlängelte Linien α- und β-Konfiguration sowie R und $R_1$ folgende Reste bedeuten.

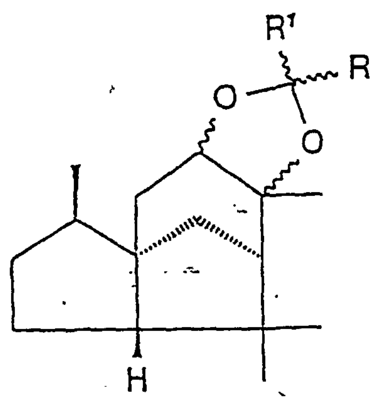

(A)

R = $R_1$ = H
R = H, $R_1$ = Me/R = Me, $R_1$ = H
R = H, $R_1$ = Et/R = Et, $R_1$ = H
R = H, $R_1$ = Pr/R = Pr, $R_1$ = H
R = H, $R_1$ = Bu / R = Bu, $R_1$ = H
R=H, $R_1$ = Iso-Bu/R = Iso-Bu, $R_1$ = H
R = H, $R_1$ = Pentyl/R = Pentyl, $R_1$ = H
R = H, $R_1$ = Iso-Pentyl / R = Iso-Pentyl, $R_1$ = H
R = H, $R_1$ = Hexyl/R = Hexyl, $R_1$ = H

R = $R_1$ = Me (α,β)
R = Me, $R_1$ = Et / R = Et, $R_1$ = Me
R = $R_1$ = Et (α,β)
R = Me, R1 = Pr / R = Pr, R1 = Me
R=Et, R1 =Pr / R=Pr, R1=Et
R = R1 = Cyclobutyl
R = R1 = Cyclopentyl
R = R1 = Cyclohexyl

2. Verfahren zur Herstellung der allgemeinen Formel **A**, **dadurch gekennzeichnet, daß** man aus Cedren erhaltenes Cedrenepoxid, je nach Wahl der Reaktionsbedingungen, zu einem Gemisch der epimeren Cedrendiole oder einzeln zu einem Gemisch der diastereomeren Cedrendiole **(3a/b)** umsetzt und diese mit aliphatischen Carbonylverbindungen unter säurekatalysierten Bedingungen in Substanz oder aprotischen Lösungsmitteln unter wasserbindenden Mitteln umsetzt.

3. Verwendung der Verbindung der allgemeinen Formel **A** als Riechstoff oder Bestandteil von Riechstoffmischungen bzw. Parfümölen zur Parfümierung von kosmetischen oder technischen Gebrauchsgüten.

## Claims

1. Cyclic cedrene acetals of the general formula A, in which the wavy lines represent α- and β- configurations, and R and $R_1$ are the radicals below.

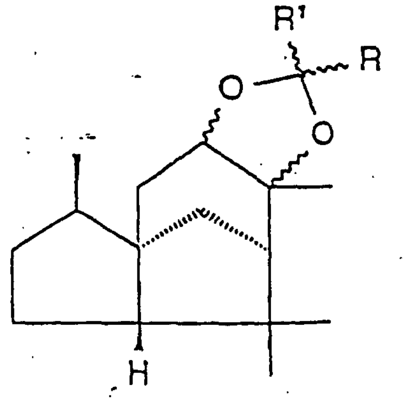

(A)

R = $R_1$ = H
R = H, $R_1$ = Me / R = Me, $R_1$ = H
R = H, $R_1$ = Et / R = Et, $R_1$ = H
R = H, $R_1$ = Pr / R = Pr, $R_1$ = H
R = H, $R_1$ = Bu / R = Bu, $R_1$ = H
R = H, $R_1$ = Iso-Bu / R = Iso-Bu, $R_1$ = H
R = H, $R_1$ = Pentyl / R = Pentyl, $R_1$ = H
R = H, $R_1$ = Isopentyl / R = Isopentyl, $R_1$ = H
R = H, $R_1$ = Hexyl / R = Hexyl, $R_1$ = H

R = $R_1$ = Me (α, β)
R = Me, $R_1$ = Et / R = Et, $R_1$ = Me
R = $R_1$ = Et (α, β)
R = Me, R1 = Pr / R = Pr, R1 = Me
R = Et, R1 = Pr / R = Pr, R1= Et
R = R1 = Cyclobutyl
R = R1 = Cyclopentyl
R = R1 = Cyclohexyl

2. Process for the preparation of the general formula **A**, **characterised in that** cedrene epoxide, obtained from cedrene, is converted into, depending on the choice of reaction conditions, a mixture of the epimeric cedrene diols or, individually, a mixture of the diastereomeric cedrenediols **(3a/b),** and the latter are reacted with aliphatic carbonyl compounds under acidcatalysed conditions in substance or aprotic solvents with the addition of water-binding agents.

3. Use of the compound of the general formula **A** as a fragrance or constituent of fragrance mixtures or perfume oils for perfuming cosmetic or industrial articles.

**Revendications**

1. Acétals de cédrène cycliques de formule générale **A**, les lignes sinueuses de configuration α et β ainsi que R et $R_1$ représentant les radicaux suivants.

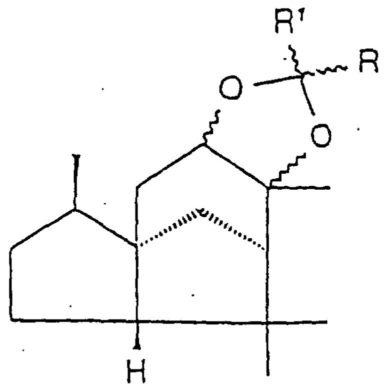

(A)

$R = R_1 = H$
$R = H, R_1 = Me/R = Me, R_1 = H$
$R = H, R_1 = Et/R = Et, R_1 = H$
$R = H, R_1 = Pr/R = Pr, R_1 = H$
$R = H, R_1 = Bu/R = Bu, R_1 = H$
R = H, $R_1$ = Iso-Bu/R = Iso-Bu, $R_1$ = H
R = H, $R_1$ = Pentyle/R = Pentyle, $R_1$ = H
R = H, $R_1$ = Iso-Pentyle/R = Iso-Pentyle, $R_1$ = H
R = H, $R_1$ = Hexyle/R = Hexyle, $R_1$ = H

$R = R_1 = Me(\alpha,\beta)$
$R = Me, R_1 = Et/R = Et, R_1 = Me$
$R = R_1 = Et(\alpha,\beta)$
$R = Me, R_1 = Pr/R = Pr, R_1 = Me$
$R = Et, R_1 = Pr/R = Pr, R_1 = Et$
R = $R_1$ = cyclobutyle
R = $R_1$ = cyclopentyle
R = $R_1$ = cyclohexyle

2. Procédé de préparation de formule générale **A**, **caractérisé en ce que** l'on fait réagir de l'époxyde de cédrène obtenu à partir du cédrène, en fonction du choix des conditions de réaction, en un mélange de cédrène-diols épimères ou individuellement en un mélange de cédrène-diols diastéréomères **(3a/b)** et **en ce que** l'on fait réagir ceux-ci dans des produits se liant à l'eau avec des composés carbonyle aliphatiques dans des conditions de catalysation acide en une substance ou des solvants aprotiques.

3. Utilisation du composé de formule générale **A** comme parfum ou ingrédient de mélanges de parfums ou d'essences de parfums pour parfumer des biens de consommation industriels ou cosmétiques.